(19) Europäisches Patentamt / European Patent Office / Office européen des brevets

(11) **EP 3 546 021 A1**

(12) **EUROPEAN PATENT APPLICATION**

(43) Date of publication:
**02.10.2019 Bulletin 2019/40**

(51) Int Cl.:
**A61N 5/10** (2006.01)    **A61B 6/02** (2006.01)

(21) Application number: **18164832.0**

(22) Date of filing: **29.03.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventors:
• **HEESE, Harald Sepp**
  **5656 AE Eindhoven (NL)**

• **VIK, Torbjoern**
  **5656 AE Eindhoven (NL)**
• **WEESE, Rolf Jürgen**
  **5656 AE Eindhoven (NL)**
• **ISOLA, Alfonso Agatino**
  **5656 AE Eindhoven (NL)**
• **NEUKIRCHEN, Christoph**
  **5656 AE Eindhoven (NL)**
• **BAL, Matthieu Frédéric**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **PLANNING SYSTEM FOR PLANNING A RADIATION THERAPY PROCEDURE**

(57)     The invention relates to a planning system 7 for planning a radiation therapy procedure, wherein the radiation therapy procedure includes rotating a radiation source 2, which is configured to emit a radiation beam 3 for treating a subject 4, along an arc around the subject, in order to apply the radiation beam to the subject in different treatment directions. A sequence of sub-arcs groups is provided, wherein a respective sub-arcs group comprises several sub-arcs of the arc, which are evenly distributed along the arc, wherein a treatment plan is generated by sequentially determining for each sub-arcs group treatment parameters, which define a property of the radiation beam, in the provided sequence. Determining the treatment parameters by using this sequence leads to an increased likelihood that really an optimal treatment plan is generated.

FIG. 1

**Description**

FIELD OF THE INVENTION

The invention relates to a planning system, method and computer program for planning a radiation therapy procedure. The invention relates further to a radiation therapy system comprising the planning system.

BACKGROUND OF THE INVENTION

The article "Volumetric modulated arc therapy: IMRT in a single gantry arc" by K. Otto, Medical Physics, volume 35, pages 310 to 317 (2007) discloses a volumetric modulated arc therapy (VMAT) system comprising a radiation source for emitting a radiation beam for treating a subject and a rotating unit for rotating the radiation source around the subject, in order to apply the radiation beam to the subject in different directions. The radiation source comprises a multi leaf collimator (MLC) for dynamically shaping the radiation beam, while the rotating unit rotates around the subject and the radiation source continuously emits the radiation beam. The radiation source and the rotating unit are controlled by a controller in accordance with a treatment plan comprising treatment parameters like, for instance, for different positions of the radiation source relative to the subject different positions of the leafs of the MLC. The treatment plan is generated by using an optimization algorithm, which tries to minimize deviations between a simulated dose distribution within the subject, which is simulated based on the treatment parameters to be determined, and a desired dose distribution within the subject. This known generation of the treatment plan has the drawback that the optimization algorithm might not find the optimal treatment parameters as it typically stops when no significant improvement can be obtained anymore. Moreover, the known optimization algorithm tends to iterate towards treatment parameter solutions being very noisy, i.e., for instance, having strong oscillations.

SUMMARY OF THE INVENTION

It is an object of the present invention to provide a planning system, method and computer program, which allow for an improved planning of a radiation therapy procedure. It is a further object of the present invention to provide a radiation therapy system comprising the planning system.

In a first aspect of the present invention a planning system for planning a radiation therapy procedure is presented, wherein the radiation therapy procedure includes rotating a radiation source, which is configured to emit a radiation beam for treating a subject, along at least one arc around the subject, in order to apply the radiation beam to the subject in different treatment directions, wherein the planning system comprises:

- a sequence providing unit configured to provide a sequence of sub-arcs groups, wherein a respective sub-arcs group comprises several sub-arcs of the at least one arc, which are evenly distributed along the at least one arc,
- a treatment plan generating unit configured to generate a treatment plan by sequentially determining for each sub-arcs group treatment parameters, which define a property of the radiation beam, in the provided sequence.

Generally, while determining treatment parameters, i.e. while applying an optimization algorithm for generating a treatment plan, dosimetric correlations between opposing or adjacent radiation beam directions can lead to the effect that for different configurations of treatment parameters equivalent results are obtained. The determined treatment plan might therefore not comprise the optimal set of treatment parameters. Moreover, the dosimetric correlation between opposing or adjacent radiation beam directions can also lead to very noisy treatment parameter solutions, i.e., for instance, to strong oscillations in the treatment parameter solutions. However, if the arc, along which the radiation source is moved during the treatment, is subdivided into sub-arcs and if these sub-arcs are grouped such that in each resulting sub-arcs group the sub-arcs are distributed as even as possible, a sequence can be provided, which leads to a significant reduction in dosimetric correlation between opposing or adjacent beam directions, if the treatment plan is generated sequentially in accordance with this sequence. This reduction of the dosimetric correlation between the opposing or adjacent beam directions leads to an increased likelihood that really the optimal configuration of treatment parameters and hence the optimal treatment plan is determined, because different treatment parameter configurations lead less likely to equivalent planning solutions. Furthermore, the reduction of the effect of dosimetric correlations between opposing or adjacent beam directions also leads to a reduction of the noisiness of the solutions. Thus, by sequentially determining for each sub-arcs group the treatment parameters in accordance with the generated sequence, the quality of the determined treatment plan can be increased.

Preferentially, the sequence providing unit is further configured to provide the sequence such that a beam direction similarity measure being indicative of a degree of beam direction similarity of radiation beams corresponding to neighboring sub-arcs groups for all sub-arcs groups in the sequence is reduced in comparison to the beam direction similarity measure for another sequence of the sub-arcs groups. This beam direction similarity measure can also be regarded as being an overall beam direction similarity measure, because it considers all pairs of neighboring sub-arcs groups in the sequence. Thus, among all possible sequences of the sub-arcs groups a sequence is provided for which the beam direction similarity measure is not maximized. Preferentially, the provided sequence belongs to the best 20 percent of all possible sequences of the sub-arcs groups with respect to the criterion of having a small degree of beam direction similarity of radiation beams corresponding to neighboring sub-arcs groups in the sequence. It is further preferred that the provided sequence belongs to the best 10 percent and it is even further preferred that it belongs to the best 5 percent. In a preferred embodiment the provided sequence is a sequence for which the beam direction similarity measure is minimal. Thus, the sequence providing unit can be configured to provide the sequence such that the beam direction similarity measure is minimized for the provided sequence. This leads to a further reduction in dosimetric correlation between opposing or adjacent beam directions and hence to a further improved quality of the treatment plan.

The treatment plan is preferentially a treatment plan to be used by a radiation therapy system comprising a radiation source for emitting the radiation beam for treating the subject, a rotating unit like a rotating gantry for rotating the radiation source around the subject, in order to apply the radiation beam to the subject in different directions, and a controller for controlling the radiation source and the rotating unit in accordance with the planned radiation therapy procedure. The radiation source preferentially comprises an MLC for shaping the radiation beam as desired. In particular, the radiation therapy system is a VMAT system.

The planning system preferentially further comprises a constraints providing unit configured to provide constraints for the treatment plan to be generated, which are defined by a radiation therapy system with which the treatment plan should be applied to the subject, wherein the treatment plan generating unit is configured to generate the treatment plan under consideration of the provided constraints. The constraints are, for instance, a possible maximum speed of moving leafs of an MLC of the radiation source, possible intensities of the radiation beam, possible positions of the leafs of the MLC, et cetera. The treatment plan generating unit is preferentially configured such that only treatment parameters are allowed for defining the treatment plan, which are in accordance with the provided constraints of the radiation therapy system. This ensures that the generated treatment plan can really be applied to the subject without any further modifications of the treatment plan which could reduce its quality.

Moreover, preferentially the planning system comprises a desired dose distribution providing unit configured to provide a desired dose distribution within the subject, wherein the treatment plan generating unit comprise a relation between the treatment parameters to be determined and a simulated dose distribution within the subject, wherein the treatment plan generating unit is configured to determine the treatment parameters such that a deviation measure being indicative of a deviation of the simulated dose distribution and the desired dose distribution is minimized. This ensures that the generated treatment plan leads to a dose distribution within the subject, which is as close as possible to the desired dose distribution.

The treatment plan generating unit is preferentially further configured to determine for each sub-arcs group a respective simulated sub dose distribution based on the treatment parameters determined for the respective sub-arcs group and to determine the simulated dose distribution by combining the simulated sub dose distributions determined for the sub-arcs groups. In particular, the treatment plan generating unit is configured to, while determining the treatment parameters and hence a simulated sub dose distribution for a respective sub-arcs group, not modify at least some of the treatment parameters determined for other sub-arcs groups. Moreover, the treatment plan generating unit can be configured to, while determining the treatment parameters and hence a simulated sub dose distribution for a respective sub-arcs group, not modify a first class of the treatment parameters determined for the other sub-arcs groups, but to modify a second class of the treatment parameters determined for the other sub-arcs groups. The radiation source preferentially includes a collimator with collimator leafs for collimating the radiation beam, wherein in an embodiment the first class of the treatment parameters includes the positions of the collimator leafs relative to the radiation beam and the second class of treatment parameters includes the intensity of the radiation beam. It has been found that these ways of considering the generated sequence of sub-arcs groups lead to a further improved quality of the generated treatment plan.

Preferentially the sequence providing unit is configured to provide the sequence of sub-arcs groups such that each sub-arcs group comprises an uneven number of sub-arcs. This can lead to a further reduction of adverse effects of dosimetric correlations and hence to less noisy treatment parameter solutions, which turn can lead to a further increased quality of the determined treatment plan.

The treatment plan generating unit is preferentially adapted to determine the treatment parameters iteratively, wherein in an iteration step the determination of the treatment parameters is started with the treatment parameters obtained in the previous iteration step. Preferentially, in an iteration step the treatment parameters for each sub-arcs group of the provided sequence are determined, wherein within a same iteration step the treatment parameters for the different sub-arcs groups are sequentially determined in accordance with the provided sequence. In an embodiment the sequence providing unit is configured to provide different sequences for different iteration steps, wherein the treatment plan generating unit is configured to, in a respective iteration step, use the sequence provided for the respective iteration step. It has been found that by using such an iteration the quality of the finally generated treatment plan can be further improved.

In a further aspect of the present invention a radiation therapy system is presented, wherein the radiation therapy system comprises:

- a radiation source configured to emit a radiation beam for treating a subject,
- a rotating unit configured to rotate the radiation source around the subject, in order to apply the radiation beam to the subject in different directions,
- a planning system for planning a radiation therapy procedure as defined by claim 1,
- a controller for controlling the radiation source and the rotating unit in accordance with the planned radiation therapy procedure.

In another aspect of the present invention a planning method for planning a radiation therapy procedure is presented, wherein the radiation therapy procedure includes rotating a radiation source, which is configured to emit a radiation beam for treating a subject, along at least one arc around the subject, in order to apply the radiation beam to the subject in different treatment directions, wherein the planning method comprises:

- providing a sequence of sub-arcs groups by a sequence providing unit, wherein a respective sub-arcs group comprises several sub-arcs of the at least one arc, which are evenly distributed along the at least one arc,
- generating a treatment plan by sequentially determining for each sub-arcs group treatment parameters, which define a property of the radiation beam, in the provided sequence by a treatment plan generating unit.

In a further aspect of the present invention a planning computer program for planning a radiation therapy procedure is presented, wherein the computer program comprises program code means for causing a planning system as defined in claim 1 to carry out the steps of the planning method as defined in claim 13, when the computer program is run on a computer controlling the planning system.

It shall be understood that the planning system of claim 1, the radiation therapy system of claim 12, the planning method of claim 13, and the planning computer program of claim 14 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplarily an embodiment of a radiation therapy system,
Fig. 2 shows schematically and exemplarily an embodiment of a planning system for planning a radiation therapy procedure,
Fig. 3 illustrates schematically and exemplarily sub-arcs of different sub-arcs groups, and
Fig. 4 shows a flowchart exemplarily illustrating an embodiment of a planning method for planning a radiation therapy procedure.

DETAILED DESCRIPTION OF EMBODIMENTS

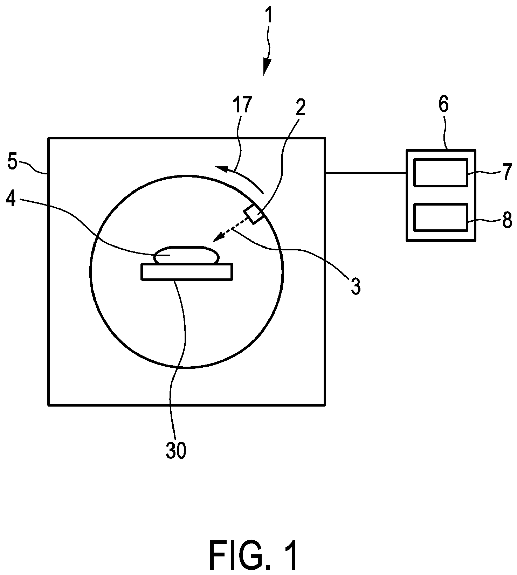

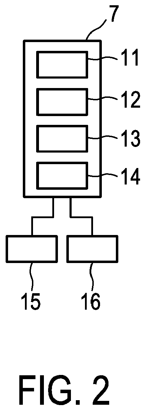

Fig. 1 shows schematically and exemplarily an embodiment of a radiation therapy system being a VMAT system. The VMAT system 1 comprises a radiation source 2 configured to emit a radiation beam 3 for treating a subject 4. In this embodiment the subject 4 is a human patient lying on a support means 30 like a patient table. In another embodiment the subject can also be an animal. The radiation source 2 is attached to a gantry 5 configured to rotate the radiation source 2 around the subject 4 in the direction indicated by the arrow 17, in order to apply the radiation beam 3 to the subject 4 in different directions. Moreover, the radiation source 2 comprises an MLC for shaping the radiation beam 3. The VMAT system 1 further comprises a control system 6 with a planning system 7 for planning a radiation therapy procedure to be carried out by the VMAT system 1 and a controller 8 for controlling at least the radiation source 2 and the gantry 5 in accordance with the planned radiation therapy procedure. The controller 8 can also be adapted to control further parts of the VMAT system 1 like the support means 30. The planning system 7 is schematically and exemplarily illustrated in Fig. 2.

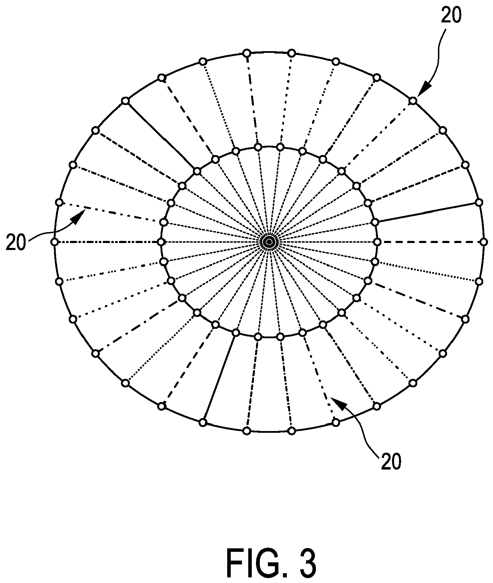

The planning system 7 comprises a sequence providing unit 11 configured to provide a sequence of sub-arcs groups, wherein the respective sub-arcs group comprises several sub-arcs of an arc along which the radiation source 2 is moved relative to the subject 4. The sub-arcs groups are formed such that in each sub-arcs group the sub-arcs are angularly distributed as even as possible. This will in the following be explained with reference to Fig. 3.

In Fig. 3 an arc of 360 degrees is assumed, wherein this arc is subdivided into sub-arcs of 12 degrees and wherein each sub-arc is represented by a respective line between two circles, wherein each line represents a central radiation beam of the respective sub-arc. In this example the resulting 30 sub-arcs are grouped into 10 groups, wherein each group contains three sub-arcs. In Fig. 3 sub-arcs belonging to the same sub-arcs group are indicated by the same kind of line type. As can be seen in Fig. 3, the sub-arcs of a same group are angularly distributed as even as possible, i.e. the angular distance between neighboring sub-arcs of a same group is constant. For instance, the sub-arcs 20 belong to the same sub-arcs group and between two neighboring sub-arcs 20 of this sub-arcs group the angular distance is 120 degrees. This distribution of sub-arcs within a same sub-arcs group leads to the effect that in each sub-arcs group the sub-arcs are neither adjacent nor opposing each other. The sequence providing unit 11 is further configured to provide the sequence of sub-arcs groups such that the sub-arcs groups are ordered in a certain way, i.e. such that a beam direction similarity measure being indicative of a degree of beam direction similarity of radiation beams corresponding to neighboring sub-arcs groups in the sequence is minimized. For instance, for ordering the sub-arcs groups, starting from an arbitrary sub-arc group, a next sub-arc group in the sequence can be determined as the one with minimum *"average minimum angular distance"* with respect to the previous sub-arcs group being rotated by 40 degrees, wherein the *"average minimum angular distance"* can be defined by following equation:

$$\frac{1}{|I|} \sum_{i \in I} \min_{j \in J} d_{360}(i,j) \qquad ,$$

wherein *I* and *J* indicate the two sub-arcs groups for which the *"average minimum distance"* should be determined and wherein $d_{360}(i,j)$ is the angle between the respective central radiation beams of the *i*-th sub-arc of the sub-arcs group *I* and the *j*-th sub-arc of the sub-arcs group *J*. Regarding this angle, following is defined:

$$0 \leq d_{360}(i,j) < 180$$

The choice of 40 degrees is a decent approximate that yields a scheme, which is not greedy, but gives good control over the maximum value of all consecutive pairs.

Thus, in an embodiment the sequence providing unit 11 is configured to start with an arbitrary sub-arcs group and then to rotate this arbitrary sub-arcs group by 40 degrees. Then, from the remaining sub-arcs groups, i.e. from all sub-arcs groups excluding the arbitrary sub-arcs group, the sub-arcs group is selected which has the smallest average minimum distance to the rotated arbitrary sub-arcs group. The arbitrary sub-arcs group is the first sub-arcs group in the sequence and the sub-arcs group with the smallest average minimum distance to the rotated first sub-arcs group is the second sub-arcs group in the sequence. Then, the second sub-arcs group is rotated by 40 degrees and among the remaining sub-arcs groups, i.e. among all sub-arcs groups excluding the first sub-arcs group and the second sub-arcs group, the sub-arcs group is determined which has the smallest average minimum distance to the rotated second sub-arcs group. This sub-arcs group having the smallest average minimum distance to the rotated second sub-arcs group is the third sub-arcs group in the sequence. This procedure is repeated, until all sub-arcs groups are ordered in the sequence. It has been found that, if the sequence is generated in this way, the average minimum distance between subsequence sub-arcs groups in the sequence has a relatively small variation, i.e., for instance, the difference between a smallest average minimum distance and a largest average minimum distance is relatively small. In this sense the suggested way of generating the sequence provides a good control over the maximum average minimum distance of all consecutive pairs of sub-arcs groups in the sequence. The sequence providing unit 11 is therefore also preferentially configured to provide the sequence of sub-arcs groups such that a variation of the average minimum distance of subsequent sub-arcs groups in the sequence is reduced, i.e., for instance, smaller than for one or more other possible sequences. In an embodiment, the sequence providing unit 11 is configured such that this variation is smaller than a predefined variation threshold and/or minimized.

Instead of a rotation angle of 40 degrees also a rotation angle of 320 degrees, i.e. of minus 40 degrees, leads equivalently to good results. Although the rotation angle of 40 degrees and the rotation angle of minus 40 degrees or 320 degrees are preferred, in another embodiment also another rotation angle can be used.

The sum or another combination of the average minimum distances determined for all pairs of neighboring sub-arcs groups in the sequence can be regarded as being a beam direction similarity measure for the sequence, wherein due to the rotation by 40 degrees it can be ensured that this beam direction similarity, which can also be regarded as being an overall beam direction similarity, is relatively low.

The planning system 7 further comprises a treatment plan generating unit 14 configured to generate a treatment plan by sequentially determining for each sub-arcs group treatment parameters which define a property of the radiation beam 3 in the generated sequence. The planning system 7 further comprises a constraints providing unit 12 configured to provide constraints for the treatment plan to be generated, which are defined by the radiation therapy system 1 with which the treatment plan should be applied to the subject 4, wherein the treatment plan generating unit 14 is configured to generate the treatment plan under consideration of the provided constraints. Moreover, the planning system 7 comprises a desired dose distribution providing unit 13 configured to provide a desired dose distribution within the subject 4, wherein the treatment plan generating unit 14 comprises a relation between the treatment parameters to be determined and a simulated dose distribution within the subject 4 and wherein the treatment plan generating unit 14 is configured to determine the treatment parameters such that a deviation measure being indicative of a deviation of the simulated dose distribution and the desired dose distribution is minimized. The desired dose distribution is preferentially defined with respect to a planning image showing a target region like a tumor within the subject to be treated and at least the surrounding of the target region, in which one or several organs at risk might be present.

The desired dose distribution can define, for instance, that a tumor region should receive a certain homogeneous target dose. A corresponding deviation measure could be defined as follows:

$$\sum_{v \in T} w_v \frac{(d_v^{sim} - d_v^{des})^2}{(d_v^{des})^2} \qquad , \qquad (1)$$

wherein v indicates a voxel of a voxelized representation of a tumor region T, $w_v$ indicates a voxel-specific weight which might be, for instance, a relative volume of the voxel, i.e. the volume fraction of the voxel, which belongs to the tumor, $d_v^{des}$ indicates the desired target dose in the respective voxel and $d_v^{sim}$ indicates the simulated dose in the respective voxel. The desired dose distribution can also define that in a nearby risk region the dose should not exceed a predefined maximum. A corresponding deviation measure could be defined as follows:

$$\sum_{v \in R} w_v \frac{H(d_v^{sim} - d_v^{des})(d_v^{sim} - d_v^{des})^2}{(d_v^{des})^2} \qquad , \qquad (2)$$

wherein R denotes a voxelized risk region and H is the Heaviside function which can be defined as follows:

$$H(x) = \begin{cases} 1, & x > 0 \\ 0, & else \end{cases} . \qquad (3)$$

These deviation measures may also be generalized by using, for instance, the negative Heaviside function. It is also possible to restrict these deviation measures to sub regions of, for instance, the tumor and/or the risk region.

In this embodiment the treatment plan generating unit 14 is configured to determine for each sub-arcs group a respective simulated sub dose distribution based on the treatment parameters determined for the respective sub-arcs group and to determine the simulated dose distribution by combining the simulated sub dose distributions determined for the sub-arcs groups. This combination is preferentially a summation of the simulated sub dose distributions.

The treatment plan generating unit 14 is further configured to, while determining the treatment parameters and hence a simulated sub dose distribution for a respective sub-arcs group, not modify at least some of the treatment parameters determined for the other sub-arcs groups. For instance, the treatment plan generating unit 14 can be configured to, while determining the treatment parameters and hence a simulated sub dose distribution for a respective sub-arcs group, not modify a first class of the treatment parameters determined for the other sub-arcs groups, but to modify a second class of the treatment parameters determined for the other sub-arcs groups. In this embodiment the first class of the treatment parameters are geometry-related parameters like the positions of the collimator leafs of the MLC relative to the radiation beam 3 and the second class of treatment parameters includes the intensity of the radiation beam 3 and/or the rotating speed of the gantry 5, wherein these treatment parameters might be regarded as being weight-related treatment parameters.

Moreover, the treatment plan generating unit 14 is adapted to determine the treatment parameters iteratively, wherein in an iteration step the determination of the treatment parameters is started with the treatment parameters obtained in the previous iteration step. Preferentially, in an iteration step the treatment parameters for each sub-arcs group of the provided sequence are determined, wherein within a same iteration step the treatment parameters for the different sub-arcs groups are sequentially determined in accordance with the provided sequence. Thus, after an iteration, all treatment parameters for all sub-arcs groups have been updated. Moreover, the sequence providing unit 11 can be configured to provide different sequences for different iteration steps, wherein the treatment plan generating unit 14 can be configured to, in a respective iteration step, use the sequence provided for the respective iteration step.

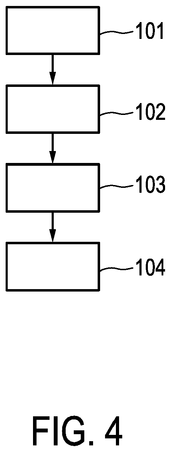

In the following an embodiment of a planning method for planning a radiation therapy procedure will exemplarily be described with reference to a flowchart shown in Fig. 4.

In step 101 the sequence of sub-arcs groups is provided by the sequence providing unit 11, wherein a respective sub-arcs group comprises several sub-arcs of the arc, which are angularly evenly distributed along the arc, and the sub-arcs groups are ordered such that a beam direction similarity measure being indicative of a degree of beam direction similarity of radiation beams corresponding to neighboring sub-arcs groups in the sequence is minimized.

In step 102 the constraints providing unit 12 provides the constraints for the treatment plan to be generated, which are defined by the radiation therapy system 1 with which the treatment plan should be applied to the subject 4, and in step 103 the desired dose distribution providing unit 13 provides a desired dose distribution within the subject 4.

In step 104 the treatment plan generating unit 14 generates the treatment plan by sequentially determining for each sub-arcs group treatment parameters, which define their property of the radiation beam 3, in the generated sequence, wherein the treatment parameters are determined such that the deviation measure being indicative of a deviation of the simulated dose distribution and the desired dose distribution is minimized. Moreover, the treatment plan generating unit 14 considers the provided constraints during the determination of the treatment parameters such that the determined treatment parameters are in accordance with the constraints. The generated treatment plan can then be provided to the controller 8 of the radiation therapy system 1, which controls the radiation therapy system 1 in accordance with the generated treatment plan.

The sequence of steps 101 to 103 can also be different. For instance, step 103 can be performed before steps 101 and 102. Moreover, in step 104 the treatment plan is preferentially generated iteratively, wherein in each iteration step the same sequence of sub-arcs groups can be used or different sequences of sub-arcs groups can be used in the different iteration steps.

The treatment parameters, which are optimized, i.e. determined, during the generation of the treatment plan, include preferentially the intensity of the radiation beam and the positions of the leafs of the MLC of the radiation source for shaping the radiation beam 3. Further treatment parameters can be, for instance, the rotation speed of the gantry 5 and/or the energy of the radiation beam 3. The treatment parameters can be dynamically modulated while the radiation beam 3 continuously irradiates the subject 4.

The trajectory, along which the radiation source 2 is rotated around the subject 4, is called an "arc", wherein during this movement along the arc the radiation source including the MLC will be controlled according to the determined treatment parameters, wherein this control in accordance with the treatment parameters can lead to a change of, for instance, the configuration of the radiation source 2 in quick succession, wherein the treatment parameters and hence possible changes in configuration are determined for several positions along the arc, which can be called "control points". Correspondingly, the planning system 7, particularly the treatment plan generating unit 14, is preferentially configured to determine treatment parameters for each control point along the arc.

The planning system 7 can be configured to provide, for instance, a graphical user interface allowing a user via an input unit 15 like a keyboard, a computer mouse, a touchpad, et cetera and a display 16 to define one or several arcs, i.e., one or several trajectories along which the radiation source 2 is continuously rotated around the subject 4, wherein each arc is described by a discrete set of control points. The treatment parameters are determined for these control points, while for the transitions between the control points the treatment parameters might be modeled by using piecewise linear or piecewise constant models.

The determination of the treatment parameters for the different control points is an inverse problem which is solved by using an optimization algorithm which tries to determine the treatment parameters for the different control points such that the resulting simulated dose distribution matches as good as possible a desired dose distribution. For determining the treatment parameters known optimization algorithms can be used like the optimization algorithm disclosed in WO 2016/046683 A2 which is herewith incorporated by reference. In particular, the treatment parameters can be determined as described in connection with the "predictor-corrector" optimizer which generates MLC leaf positions from a predicted dose which in turn is obtained by optimization of a current approximate dose from a set of control points. Also other known optimization algorithms can be used for determining the treatment parameters.

The desired dose distribution can simply be, for instance, that only a target like a tumor should receive a therapeutic dose, i.e. a dose being larger than a predefined dose threshold. A corresponding deviation from this desired dose distribution could be defined by the so called "conformity index". The desired dose distribution can also define, for instance, a first dose which should be received by a first percentage of the target and a second dose which should be received by a second percentage of the target, wherein this desired dose distribution could be defined by a homogeneity index, wherein the treatment parameters can be optimized such that the desired homogeneity index is achieved.

The generated treatment plan is preferentially a VMAT treatment plan. VMAT treatment plans have the advantage that they are relatively fast to deliver as the radiation beam is used in a continuous irradiation mode. The VMAT treatment plan can also lead to a very high conformity with the desired dose distribution, especially in comparison to step-and-shoot treatment plans, because the beam energy is smeared out across a larger angular range. For this reason, the respective arc can be relatively long, i.e., for instance, larger than 180 degrees, and can even be 360 degrees.

The treatment parameters, which can be modulated during radiation delivery, can include, as mentioned above, leaf positions of the MLC, the dose rate, i.e. the intensity of the radiation beam, the gantry speed, the energy of the radiation beam, et cetera. The treatment parameters can also include positions of jaws of the radiation source 2. In particular, the radiation source 2 comprises a linear accelerator for generating the initial radiation beam, i.e. for generating the radiation beam before collimation, wherein in addition to the MLC, the radiation source 2 comprises a further collimation system comprising several jaws, wherein each jaw can block a side of the initial radiation beam. Each jaw can be moved in or out, in order to block a larger or smaller portion at the respective side of the radiation beam. Preferentially, the radiation source 2 has four jaws for blocking a portion of the radiation beam 3 on the left, on the right, on the top and at the bottom. In this way the "field" of the radiation beam 3 can be delimited. This field is then further collimated with the more complex structure of the leafs of the MLC.

The use of relatively long VMAT arcs adds geometric complexity to the determination of the treatment parameters as dosimetric effects of opposing beam directions are strongly correlated. The performance of mathematical optimization algorithms, i.e. of the determination of the treatment parameters, typically suffers in such situations in the sense that different treatment parameter configurations can lead to equivalent results. Such behavior can have the negative side effect that mathematical optimizations fall short of the actual optimal configuration of treatment parameters. Another typical adverse behavior is that optimization algorithms iterate towards states that are very noisy, i.e., for instance, that have strong oscillations. Such behavior is a consequence of the strong dosimetric correlation between the opposing or adjacent beam directions in known planning schemes. These adverse effects are overcome or at least reduced by determining the treatment parameters for each sub-arcs group in accordance with the provided sequence as explained above and as it will also be further explained in the following.

The determination of the treatment parameters sequentially for each sub-arcs group in accordance with the provided sequence can be regarded as being a sequential subset optimization strategy of control points which prevents geometric instabilities from, for instance, opposing beam directions and which stabilizes the performance of the optimization algorithm by limiting the amount of free parameters. In particular, one or several VMAT arcs can be defined by, for instance, the user, wherein each VMAT arc is subdivided into a number of sub-arcs. The length of these sub-arcs may be, for instance, 12 degrees, 20 degrees or 52 degrees, wherein the number of these sub-arcs is chosen accordingly and wherein, if the total length of the arc does not match to the subdivision, the last sub-arc is adjusted to the total length of the arc. The collection of sub-arcs is rearranged into sub-arcs groups of, for instance, 3, 5, 7 or 9 sub-arcs, wherein the sub-arcs of a same sub-arcs group are angularly distributed along the respective VMAT arc as evenly as possible. In particular, the centers of these sub-arcs within each sub-arcs group are spaced as evenly as possible. The resulting collection of sub-arcs groups is finally ordered such that a strong correlation for successive sub-arcs groups is avoided. Thus, the sub-arcs groups are ordered such that a beam direction similarity measure being indicative of a degree of beam direction similarity of radiation beams corresponding to neighboring sub-arcs groups in the sequence is minimized. For instance, each pair of sub-arcs groups can be assessed for the correlation, wherein correlation in this context is characterized by a similarity of beam directions. The similarity of beam directions can be measured, for instance, by the planar angle between the beam directions. An ordering of the sub-arcs groups can be achieved based on this similarity measure, wherein starting from an arbitrary sub-arcs group the next one can be chosen from the remaining sub-arcs groups as the one that has a minimum value with respect to the above-described correlation.

For the optimization of the treatment plan, i.e. for the determination of the treatment parameters, the treatment plan generating unit 14 sequentially optimizes each sub-arcs group according to the provided constraints and preferentially under consideration of a fixed dosimetric contribution of the other sub-arcs groups, which is generated based on their current treatment parameters, i.e. based on the treatment parameters determined for the other sub-arcs groups during the iterative optimization procedure. The provided constraints define, for instance and as mentioned above, the maximum speed of the leafs of the MLC. As the determination of the treatment parameters, i.e. the optimization process, aims at determining, for instance, an optimal succession of leaf positions as treatment parameters at the control points, the optimization has to take the speed limits of the movement of the leafs of the MLC into account, in order to produce a realizable succession of the leaf positions. In an embodiment another constraint is that opposing leafs of adjacent leaf pairs are not allowed to move past each other, i.e. to interdigitate, in order to avoid hazard for the MLC.

In an embodiment the leaves of the MLC on a respective side are jointly mounted on a carriage, i.e. a first set of leaves is mounted to a first carriage on one side and a second set of leaves is mounted on a second carriage on a second opposing side. Each leaf is independently movable with respect to the respective carriage and also the carriage is movable. The position of the respective leaf can therefore be defined by the position of the respective leaf relative to the respective carriage and by the position of the respective carriage. Constraints can therefore be provided for the position of the respective carriage and for the position of the respective leaf relative to the respective carriage. Constraints can also be provided with respect to the speed of the respective carriage and the speed of the movement of the respective leaf with respect to the respective carriage.

The treatment plan generating unit 14 can be adapted to start the iterative determination of the treatment parameters for the different control points along the respective arc with initial treatment parameters, i.e. with an initial treatment plan, wherein this initial treatment plan is iteratively optimized during the planning procedure. The initial treatment plan can be an arbitrary treatment plan with arbitrary default values for the treatment parameters, but the initial treatment plan can also be a "good guess". For instance, the target region can be defined by a planning image showing, for instance, a tumor, wherein for each control point the leaves can initially be positioned such that they conform to the outline of the target region in the respective radiation direction defined by the respective control point. The initial treatment plan can also define initial positions of the leaves such that they conform to a rectangular approximation of the target region at the respective control point. A "good guess" can also be taken from scientific literature which refers to, for instance, "sequencing algorithms" or "conversion algorithms" in the context of VMAT. The initial treatment plan can also be provided by a user like a physician.

Preferentially, the number of sub-arcs of a same sub-arcs group is uneven, in order to ensure that opposing beam directions are not located in a same sub-arcs group. Moreover, the length of the respective sub-arc, the number of sub-arcs per sub-arcs group and the number of the sub-arcs groups are preferentially chosen such that the product of these values is equal to the arc of, for instance, 360 degrees. Based on these values a very balanced sequence of sub-arcs groups can be determined and provided, which in turn can lead to a high quality treatment plan.

Before the actual optimization algorithm starts, for each sub-arc a representative beam direction might be selected, which preferentially corresponds to the center of the respective sub-arc. Moreover, so called "optimization data" may be computed, which might later be used for the actual optimization algorithm, i.e. for deriving updates that improve the simulated dose distribution such that it better corresponds to the desired dose distribution. The optimization data describe a simulated dose from a small area around the leaf positions for a control point. For more details regarding the optimization data and their use for the optimization reference is made to the above mentioned patent document WO 2016/046683 A2, especially to the prediction step described in this patent document, which describes equivalently how the optimization data might be used. This is herewith incorporated by reference.

All control points within a respective sub-arc might be identified with respect to the representative beam direction of the sub-arc.

The treatment plan generating unit 14 is preferentially adapted to start with an initial treatment plan being, as mentioned above, a "good guess", wherein the treatment plan defines treatment parameters for different control points. These initial treatment parameters are optimized by the treatment plan generating unit, wherein this optimization of the treatment parameters is carried out sequentially per sub-arcs group in accordance with the provided sequence. In particular, the treatment plan generating unit computes for each sub-arcs group a sub dose distribution and hence corresponding treatment parameters for the respective control points. The optimization, which is carried out for the respective sub-arcs group, is based on an above mentioned deviation measure which in turn depends on a deviation between a simulated dose distribution and a desired dose distribution. The desired dose distribution is the dose distribution which should be obtained when the finally determined treatment plan is carried out for all control points. The simulated dose distribution, which is used for the optimization of the treatment parameters of a certain sub-arcs group, can be regarded as being composed of the simulated sub dose distribution of the certain sub-arcs group, which is modified by the treatment plan generating unit during the optimization of the certain sub-arcs group, and a simulated dose distribution caused by all other sub-arcs groups. The latter simulated dose distribution is preferentially fixed, if the treatment parameters and hence the simulated sub dose distribution for the certain sub-arcs group are optimized. However, as mentioned above, it is also possible that only a first class of treatment parameters is fixed for the simulated dose distributions of the other sub-arcs groups and that a second class of treatment parameters is not fixed. The treatment plan generation is preferentially carried out iteratively, as mentioned above, wherein in a single iteration step all sub-arcs groups are sequentially optimized, i.e. the respective treatment parameters are optimized, in accordance with the provided sequence.

After the treatment parameters for the control points of a respective sub-arcs group and hence the corresponding simulated dose contributions have been updated, this updating procedure continuous with the next sub-arcs group. In an embodiment not all treatment parameters for the other sub-arcs group are fixed, but the updating of the treatment parameters for a respective sub-arcs group can also lead to changes of treatment parameters of the other sub-arcs groups. Thus, a first class of treatment parameters might be fixed for the other sub-arcs groups and another, second class of treatment parameters might not be fixed, wherein this second class of treatment parameters includes, for instance, the intensity of the radiation beam. Changing the intensity of the radiation beam can be regarded as being a weighting of the control points, i.e. of the optimization. The determination of an update for a sub-arcs group may hence allow an adjustment of the weights of all control points of all sub-arcs.

The entire procedure can be iterated with the same or different design parameters, i.e. with the same or different sequences of sub-arcs groups, until a sufficient plan quality is obtained, i.e. until a deviation between the simulated overall dose distribution and the determined overall dose distribution has been minimized and is below a predefined deviation threshold. Thus, in an iteration step the treatment parameters for all sub-arcs groups can be updated, wherein this updating is carried out sequentially for the different sub-arcs groups in a same iteration step, wherein before continuing with a next iteration step, the design parameters can remain or can be modified.

If treatment parameters are to be determined for several arcs, the generation of the sequence of sub-arcs groups can consider all arcs simultaneously, i.e. all arcs can be subdivided into sub-arcs, the sub-arcs can be rearranged into groups such that in each sub-arcs group the sub-arcs are angularly evenly distributed independently of to which arc the respective sub-arc belongs to, and finally the generated sub-arcs groups can be ordered for generating the final sequence. As a result, any sub-arcs group may include elements from different initial arcs.

As explained above, the non-optimizing control points, i.e. the control points of the sub-arcs groups which are currently not updated, can only be fixed with respect to a first class of treatment parameter, which could be defined as being beam geometry related treatment parameters like leaf positions of the MLC and jaw positions, while the second class of treatment parameters, which could be defined as being weight-related parameters like the intensity of the radiation beam or the gantry speed, are allowed to change freely within the given constraints for all control points, i.e. also for the control points of the sub-arcs groups which are currently not updated.

Although in above described embodiments the planning system is adapted to plan a VMAT procedure and the radiation therapy system is a VMAT system, which refer to a continuous delivery of radiation along one or several planar arcs, wherein each planar arc covers 360 degrees or less, the planning system can also be used to plan a radiation therapy to be carried out by another radiation therapy system using radiation beams for treating a subject. For instance, instead of only planar trajectories, also arbitrary trajectories on a sphere in three-dimensional space can be considered. In this case instead of planar angles spatial angles on the sphere are to be used. For instance, in an embodiment the subject can be moved, i.e. a patient table can be moved, while the radiation source is rotated around the subject, wherein this movement can be, for instance, a tilting or shifting of the patient table. In this case the trajectory, along which the radiation source is moved relative to the subject, is non-planar. However, also in this case an arc is present being defined as the trajectory along which the radiation source is moved relative to the subject and this arc can also be subdivided into sub-arcs. Also in this case the sequence providing unit is configured to provide the sequence of sub-arcs groups, wherein a respective sub-arcs group comprises several sub-arcs of the arc, which are angularly evenly distributed along the arc. The angle can in this case be defined by the respective position on the arc. The angularly even distribution along the arc can therefore also be regarded as being a distribution in which the sub-arcs of a respective sub-arcs group are evenly, i.e. homogeneously, distributed along the arc. In any case, i.e. in the planar case and in the non-planar case, preferentially the positions of the sub-arcs of a respective sub-arcs group are evenly, i.e. homogeneously, distributed along the arc.

The planning system can also be used for planning a radiation therapy for a step-and-shoot intensity modulated radiation therapy (IMRT procedure), especially if the number of incident directions is relatively large, i.e., for instance, 15 or larger. An example of such an IMRT system, for which the planning system can generate a treatment plan, is disclosed in the article "Bridging the gap between IMRT and VMAT: Dense angular sampled and sparse intensity modulated radiation therapy (DASSIM-RT)" by R. Li et al., Medical Physics, volume 38, pages 4912 to 4919 (2001), which is herewith incorporated by reference.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the provision of the sequence, the determination of the treatment plan, et cetera, performed by one or several units or devices can also be performed by any other number of units or devices. These procedures and/or the control of the planning system in accordance with the planning method can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a planning system for planning a radiation therapy procedure, wherein the radiation therapy procedure includes rotating a radiation source, which is configured to emit a radiation beam for treating a subject, along an arc around the subject, in order to apply the radiation beam to the subject in different treatment directions. A sequence of sub-arcs groups is provided, wherein a respective sub-arcs group comprises several sub-arcs of the arc, which are evenly distributed along the arc, wherein a treatment plan is generated by sequentially determining for each sub-arcs group treatment parameters, which define a property of the radiation beam, in the provided sequence. Determining the treatment parameters by using this sequence leads to an increased likelihood that really an optimal treatment plan is generated.

**Claims**

1. A planning system for planning a radiation therapy procedure, wherein the radiation therapy procedure includes rotating a radiation source (2), which is configured to emit a radiation beam (3) for treating a subject (4), along at least one arc around the subject (4), in order to apply the radiation beam (3) to the subject (4) in different treatment directions, wherein the planning system (7) comprises:

   - a sequence providing unit (11) configured to provide a sequence of sub-arcs groups, wherein a respective sub-arcs group comprises several sub-arcs of the at least one arc, which are evenly distributed along the at least one arc,
   - a treatment plan generating unit (14) configured to generate a treatment plan by sequentially determining for each sub-arcs group treatment parameters, which define a property of the radiation beam (3), in the provided sequence.

2. The planning system as defined in claim 1, wherein the sequence providing unit (11) is further configured to provide the sequence such that a beam direction similarity measure being indicative of a degree of beam direction similarity of radiation beams corresponding to neighboring sub-arcs groups for all sub-arcs groups in the sequence is reduced in comparison to the beam direction similarity measure for another possible sequence of the sub-arcs groups.

3. The planning system as defined in claim 1, wherein the planning system (7) further comprises a constraints providing unit (12) configured to provide constraints for the treatment plan to be generated, which are defined by a radiation therapy system (1) with which the treatment plan should be applied to the subject (4), wherein the treatment plan generating unit (14) is configured to generate the treatment plan under consideration of the provided constraints.

4. The planning system as defined in claim 1, wherein the planning system (7) further comprises a desired dose distribution providing unit (13) configured to provide a desired dose distribution within the subject (4), wherein the treatment plan generating unit (14) comprise a relation between the treatment parameters to be determined and a simulated dose distribution within the subject (4), wherein the treatment plan generating unit (14) is configured to determine the treatment parameters such that a deviation measure being indicative of a deviation of the simulated dose distribution and the desired dose distribution is minimized.

5. The planning system as defined in claim 4, wherein the treatment plan generating unit (14) is configured to determine for each sub-arcs group a respective simulated sub dose distribution based on the treatment parameters determined for the respective sub-arcs group and to determine the simulated dose distribution by combining the simulated sub dose distributions determined for the sub-arcs groups.

6. The planning system as defined in claim 5, wherein the treatment plan generating unit (14) is configured to, while determining the treatment parameters and hence a simulated sub dose distribution for a respective sub-arcs group, not modify at least some of the treatment parameters determined for other sub-arcs groups.

7. The planning system as defined in claim 6, wherein the treatment plan generating unit (14) is configured to, while determining the treatment parameters and hence a simulated sub dose distribution for a respective sub-arcs group, not modify a first class of the treatment parameters determined for the other sub-arcs groups, but to modify a second class of the treatment parameters determined for the other sub-arcs groups.

8. The planning system (7) as defined in claim 7, wherein the radiation source (2) includes a collimator with collimator leafs for collimating the radiation beam (3), wherein the first class of the treatment parameters includes the positions of the collimator leafs relative to the radiation beam (3) and the second class of treatment parameters includes the intensity of the radiation beam (3).

9. The planning system as defined in claim 1, wherein the sequence providing unit (11) is configured to provide the sequence of sub-arcs groups such that each sub-arcs group comprises an uneven number of sub-arcs.

**10.** The planning system as defined in claim 1, wherein the planning system (7) is configured to plan a volumetric modulated arc therapy procedure.

**11.** The planning system as defined in claim 1, wherein the treatment plan generating unit (14) is adapted to determine the treatment parameters iteratively, wherein in an iteration step the determination of the treatment parameters is started with the treatment parameters obtained in the previous iteration step.

**12.** The planning system as defined in claim 11, wherein the sequence providing unit (11) is configured to provide different sequences for different iteration steps, wherein the treatment plan generating unit (14) is configured to, in a respective iteration step, use the sequence provided for the respective iteration step.

**13.** A radiation therapy system comprising:

- a radiation source (2) configured to emit a radiation beam (3) for treating a subject (4),
- a rotating unit (5) configured to rotate the radiation source (2) around the subject (4), in order to apply the radiation beam (3) to the subject (4) in different directions,
- a planning system (7) for planning a radiation therapy procedure as defined by claim 1,
- a controller (8) for controlling the radiation source (2) and the rotating unit (5) in accordance with the planned radiation therapy procedure.

**14.** A planning method for planning a radiation therapy procedure, wherein the radiation therapy procedure includes rotating a radiation source (2), which is configured to emit a radiation beam (3) for treating a subject (4), along at least one arc around the subject (4), in order to apply the radiation beam (3) to the subject (4) in different treatment directions, wherein the planning method comprises:

- providing a sequence of sub-arcs groups by a sequence providing unit (11), wherein a respective sub-arcs group comprises several sub-arcs of the at least one arc, which are evenly distributed along the at least one arc,
- generating a treatment plan by sequentially determining for each sub-arcs group treatment parameters, which define a property of the radiation beam (3), in the provided sequence by a treatment plan generating unit (14).

**15.** A planning computer program for planning a radiation therapy procedure, the computer program comprising program code means for causing a planning system as defined in claim 1 to carry out the steps of the planning method as defined in claim 14, when the computer program is run on a computer controlling the planning system.

FIG. 1

FIG. 2

FIG. 3

FIG. 4

Europäisches
Patentamt

European
Patent Office

Office européen
des brevets

# EUROPEAN SEARCH REPORT

Application Number

EP 18 16 4832

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/219098 A1 (MCNUTT TODD R [US] ET AL) 27 November 2003 (2003-11-27) * abstract; figures 1,2 * * paragraphs [0008], [0020] - [0039] * | 1-15 | INV. A61N5/10 A61B6/02 |
| X | US 2011/210261 A1 (MAURER JR CALVIN R [US]) 1 September 2011 (2011-09-01) * the whole document * | 1,13-15 | |
| X | US 2017/203124 A1 (RENO JILLIAN [US] ET AL) 20 July 2017 (2017-07-20) * the whole document * | 1,13-15 | |
| X | US 2017/189717 A1 (MACDONALD R LEE [CA] ET AL) 6 July 2017 (2017-07-06) * the whole document * | 1,13-15 | |
| X | US 2018/021594 A1 (PAPP DADIV [US] ET AL) 25 January 2018 (2018-01-25) * the whole document * | 1,13-15 | |
| X | US 2016/325119 A1 (YU XINSHENG CEDRIC [US]) 10 November 2016 (2016-11-10) * the whole document * | 1,13-15 | TECHNICAL FIELDS SEARCHED (IPC) A61B A61N |
| X | US 2012/020449 A1 (YAN DI [US] ET AL) 26 January 2012 (2012-01-26) * the whole document * | 1,13-15 | |
| X | WO 2017/089097 A1 (KONINKLIJKE PHILIPS NV [NL]) 1 June 2017 (2017-06-01) * the whole document * | 1,13-15 | |

The present search report has been drawn up for all claims

| Place of search | Date of completion of the search | Examiner |
|---|---|---|
| Munich | 7 June 2018 | Pereda Cubián, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 16 4832

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2018

| Patent document cited in search report | Publication date | Patent family member(s) | | Publication date |
|---|---|---|---|---|
| US 2003219098 A1 | 27-11-2003 | AT | 493171 T | 15-01-2011 |
| | | AU | 2003247392 A1 | 12-12-2003 |
| | | EP | 1511535 A1 | 09-03-2005 |
| | | JP | 4425129 B2 | 03-03-2010 |
| | | JP | 2005526578 A | 08-09-2005 |
| | | US | 2003219098 A1 | 27-11-2003 |
| | | WO | 03099380 A1 | 04-12-2003 |
| US 2011210261 A1 | 01-09-2011 | EP | 2539020 A1 | 02-01-2013 |
| | | EP | 2664360 A2 | 20-11-2013 |
| | | EP | 3195902 A1 | 26-07-2017 |
| | | US | 2011210261 A1 | 01-09-2011 |
| | | US | 2011211665 A1 | 01-09-2011 |
| | | US | 2015073256 A1 | 12-03-2015 |
| | | US | 2015131774 A1 | 14-05-2015 |
| | | US | 2016199666 A1 | 14-07-2016 |
| | | US | 2016303400 A1 | 20-10-2016 |
| | | WO | 2011106433 A1 | 01-09-2011 |
| US 2017203124 A1 | 20-07-2017 | NONE | | |
| US 2017189717 A1 | 06-07-2017 | EP | 3169398 A1 | 24-05-2017 |
| | | US | 2017189717 A1 | 06-07-2017 |
| | | WO | 2016008052 A1 | 21-01-2016 |
| US 2018021594 A1 | 25-01-2018 | CN | 107206253 A | 26-09-2017 |
| | | DE | 112016000448 T5 | 04-01-2018 |
| | | JP | 2018502655 A | 01-02-2018 |
| | | US | 2018021594 A1 | 25-01-2018 |
| | | WO | 2016116868 A1 | 28-07-2016 |
| US 2016325119 A1 | 10-11-2016 | CA | 2935418 A1 | 09-07-2015 |
| | | CN | 105916555 A | 31-08-2016 |
| | | EP | 3089787 A1 | 09-11-2016 |
| | | JP | 2017504449 A | 09-02-2017 |
| | | US | 2015190658 A1 | 09-07-2015 |
| | | US | 2016325119 A1 | 10-11-2016 |
| | | WO | 2015103564 A1 | 09-07-2015 |
| US 2012020449 A1 | 26-01-2012 | BR | 112012016558 A2 | 26-04-2016 |
| | | CA | 2785995 A1 | 14-07-2011 |
| | | CN | 102686277 A | 19-09-2012 |
| | | EP | 2521592 A1 | 14-11-2012 |
| | | JP | 2013516278 A | 13-05-2013 |
| | | US | 2012020449 A1 | 26-01-2012 |
| | | US | 2014100408 A1 | 10-04-2014 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**                EP 18 16 4832

This annex lists the patent family members relating to the patent documents cited in  the above-mentioned European search report.
The members are as contained in the European Patent Office EDP file on
The European Patent Office is in no way liable for these particulars which are merely given for the purpose of information.

07-06-2018

| Patent document cited in search report | Publication date | Patent family member(s) | Publication date |
|---|---|---|---|
| | | WO 2011084878 A1 | 14-07-2011 |
| WO 2017089097 A1 | 01-06-2017 | NONE | |

For more details about this annex : see Official Journal of the European Patent Office, No. 12/82

EPO FORM P0459

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016046683 A2

**Non-patent literature cited in the description**

- **K. OTTO.** Volumetric modulated arc therapy: IMRT in a single gantry arc. *Medical Physics,* 2007, vol. 35, 310-317

- **R. LI et al.** Bridging the gap between IMRT and VMAT: Dense angular sampled and sparse intensity modulated radiation therapy (DASSIM-RT). *Medical Physics,* 2001, vol. 38, 4912-4919